# EUROPEAN PATENT APPLICATION

(11) **EP 4 564 373 A1**
(43) Date of publication of application: **04.06.2025**
(21) Application number: 23846875.5
(22) Date of filing: 12.07.2023
(51) Int. Cl.: G16H 50/80, G16H 50/20, G16H 10/20, G16H 50/30, G16H 40/20, G06Q 10/10, G06Q 50/10

(54) **METHOD AND COMPUTING APPARATUS FOR TESTING COMMUNITY GROUP FOR INFECTIOUS DISEASE AND NOTIFYING TEST RESULTS**

(30) Priority: 29.07.2022 KR 20220094921
(71) Applicant: Seegene, Inc., Seoul 05548 (KR)
(72) Inventor: KIM, Young Wook, Seoul 04593 (KR); LEE, Seung Hun, Seoul 05811 (KR); KIM, Kwang Min, Namyangju-si Gyeonggi-do 12251 (KR)
(74) Representative: Viering, Jentschura & Partner mbB Patent- und Rechtsanwälte
(86) International application number: PCT/KR2023/009964
(87) International publication number: WO 2024/025216

(57) **Abstract**

The present invention relates to a method and computing apparatus for testing a community group for an infectious disease and notifying the test results, and relates to a method and computing apparatus for testing a community group for an infectious disease and notifying the test results so that infectious disease tests are more efficiently conducted and managed within each community group for individuals living in a group at a workplace, a church, a nursing home, or the like.

## Description

### [Technical Field]

The present invention relates to a method and computing apparatus for testing a community group for an infectious disease and notifying the test results, and specifically to a method and computing apparatus for testing a community group for an infectious disease and notifying the test results to ensure infectious disease tests are conducted and managed more efficiently within each community group for individuals living in a group at a workplace, a church, a nursing home, or the like.

### [Background Art]

Recently, infections caused by pathogens such as coronavirus have become a social problem. In particular, when the pathogens are highly contagious, there is a very high possibility that the pathogens will spread among individuals within the group or to an unspecified number of individuals in community groups such as schools, hospitals, companies, and shopping malls, causing infection.

In order to prevent infection by the pathogens, infectious disease tests such as molecular diagnostics tests are recommended for users to test for infection by the pathogens, and confirmation of infectious disease test results is required when users visit public facilities. Accordingly, test institutions issue users with negative certificates indicating that the infectious disease test results for the user are "negative".

However, since the above-described infectious disease test for the user is conducted based on a personal decision, when a user who has developed symptoms of the infectious disease avoids the infectious disease test and continues economic or social activities, it is not possible to prevent the spread of the infectious disease by the user with symptoms.

In addition, there may be asymptomatic users who have come into contact with an infected individual with an infectious disease but have not developed symptoms. The asymptomatic user may not undergo the infectious disease test because they do not show clear symptoms of an infectious disease. Even in this case, there is a problem in preventing the spread of infectious diseases as the asymptomatic user continues to engage in economic or social activities.

### [Disclosure]

### [Technical Problem]

A purpose of the present invention is to provide a method and a computing apparatus for quickly blocking spread of an infectious disease within a community group by performing an infectious disease test on a plurality of users belonging to the community group.

Another object of the present invention is to provide a method and a computing apparatus for providing information for infectious disease and quarantine management of a community group by providing a result obtained by comparing an infection index of an infectious disease of the community group to which a plurality of users belong with an infection index of another community group or an administrative district to the community group.

Another object of the present invention is to provide a method and a computing apparatus for managing an infectious disease test of each user in a community group including a plurality of users, and authenticating, when the community group is selected as an excellent infectious disease management group in response to the infectious disease test, the community group as a safe place from infectious diseases.

Another object of the present invention is to provide a method and a computing apparatus for performing an infectious disease test and displaying a certificate and location of a community group safe from an infectious disease on a digital map.

Another object of the present invention is to provide a method and a computing apparatus capable of improving accessibility to an infectious disease test for a plurality of users by allowing each user belonging to a community group to apply for the infectious disease test through a terminal owned by the user and conveniently check the test results.

However, the problem to be solved by the present disclosure is not limited to that mentioned above, and other problems to be solved that are not mentioned may be clearly understood by those of ordinary skill in the art to which the present disclosure belongs from the following description.

### [Technical Solution]

In accordance with an aspect of the present disclosure, there is provided a method for processing a request for an infectious disease test for a community group and a test result notification in a computing apparatus using a memory, a processor, and one or more programs stored on the memory and configured to be executed by the processor, the method comprising: generating, by the processor, a community group including a plurality of users; providing, by the processor, a test information for the infectious disease test to the plurality of users included in the community group; receiving, by the processor, test results of the infectious disease test conducted on the plurality of users; generating, by the processor, an integrated test result of the community group using the test results; and providing, by the processor, the integrated test result to a health manager of the community group.

In accordance with another aspect of the present disclosure, there is provided a device for processing a request for an infectious disease test for a community group and a test result notification, the device comprising: a memory configured to store at least one instruction; and a processor; wherein the at least one instruction is executed by the processor, to: generate a community group including a plurality of users; provide a test information for the infectious disease test to the plurality of users included in the community group; receive test results of the infectious disease test conducted on the plurality of users; generate an integrated test result of the community group using the test results; and provide the integrated test result to a health manager of the community group.

In accordance with another aspect of the present disclosure, there is provided a non-transitory computer-readable storage medium storing a computer program for processing a request for an infectious disease test for a community group and a test result notification, the computer program configured to perform: generate a community group including a plurality of users; provide a test information for the infectious disease test to the plurality of users included in the community group; receive test results of the infectious disease test conducted on the plurality of users; generate an integrated test result of the community group using the test results; and provide the integrated test result to a health manager of the community group.

In accordance with another aspect of the present disclosure, there is provided a computer program for processing a request for an infectious disease test for a community group and a test result notification, stored in a non-transitory computer-readable storage medium, the computer program configured to perform: generate a community group including a plurality of users; provide a test information for the infectious disease test to the plurality of users included in the community group; receive test results of the infectious disease test conducted on the plurality of users; generate an integrated test result of the community group using the test results; and provide the integrated test result to a health manager of the community group.

### [Advantageous Effects]

The features and advantages of the present invention are summarized as follows.
(1) According to the method and computing apparatus for testing the community group for the infectious disease and notifying of the test results of the present invention, it is possible to quickly and collectively process the application of the infectious disease test, collection of a sample, and reception of test results for the community group including the plurality of users.
(2) According to the method and computing apparatus for testing the community group for the infectious disease and notifying of the test results of the present invention, it is possible to generate various statistical data and infection indexes using the test results from an infectious disease test for the community group including the plurality of users and provide the statistical data and infection indexes to the community group, thereby enabling the community group to quickly and accurately establish a quarantine rule for the infectious disease.
(3) According to the method and computing apparatus for testing the community group for the infectious disease and notifying of the test results of the present invention, it is possible to easily identify an infectious disease quarantine level of the community group by comparing various statistical data and infection indexes from the infectious disease test with an administrative district or another community group.
(4) According to the method and computing apparatus for testing the community group for the infectious disease and notifying of the test results of the present invention, it is possible to provide information so that a user or customer may visit the community group with excellent infectious disease management, countermeasures, and quarantine by displaying the certificate provided after the infectious disease test for the community group on a digital map.
(5) According to the method and computing apparatus for testing the community group for the infectious disease and notifying of the test results of the present invention, when the plurality of users included in the community group perform the infectious disease test, the infectious disease test may be applied within the community group, allowing the plurality of users to receive the infectious disease test with a minimum number of procedures.

### [Description of Drawings]

FIG. 1 is a configuration diagram illustrating a computing apparatus for testing a community group for an infectious disease and notifying the test results according to the present invention.
FIG. 2 is an exemplary diagram illustrating various forms of the community groups according to the present invention.
FIG. 3 and FIG. 4 are sequence diagrams illustrating the method for testing the community group for the infectious disease and notifying the results of the test according to the present invention.
FIG. 5 is a flowchart illustrating the method for testing the community group for the infectious disease and notifying the results of the test according to the present invention.
FIG. 6 is a first exemplary diagram illustrating a method for collecting samples for the infectious disease test for the community group according to the present invention.
FIG. 7 is a second exemplary diagram illustrating the method for collecting samples for the infectious disease test for the community group according to the present invention.
FIG. 8 is a third exemplary diagram illustrating the method for collecting the samples for the infectious disease test for the community group according to the present invention.
FIG. 9 is a fourth exemplary diagram illustrating a method for collecting the samples for the infectious disease test for the community group according to the present invention.
FIG. 10 is a fifth exemplary diagram illustrating the method for collecting the samples for the infectious disease test for the community group according to the present invention.
FIG. 11 is exemplary diagrams for illustrating integrated test results using the test results according to the present invention.
FIG. 12 is an exemplary diagram illustrating the infection index using the test results according to the present invention.
FIG. 13 is an exemplary diagram illustrating the certificate provided to the community group according to the infectious disease test results according to the present invention and displaying the authenticated community group on a digital map.

### [Mode for Disclosure]

Hereinafter, the present invention will be described in more detail through embodiments with reference to the drawings of the present invention. These embodiments are only intended to specifically explain the present invention, and it will be apparent to those skilled in the art that the scope of the present invention is not limited by these embodiments according to the gist of the present invention.

In addition, when describing components of the present invention, terms such as first, second, A, B, (a), (b), (i), (ii), or the like may be used. These terms are only intended to distinguish the components from other components, and the nature, order, or sequence of the components are not limited by the terms. When it is described that a component is "connected," "coupled," or "joined" to another component, it should be understood that the component may be directly connected or joined to the other component, but another component may also be "connected," "coupled," or "joined" between each component.

The present inventors have sought to solve the problem that a test for an infectious disease such as COVID-19 is being performed by an individual. In addition, although a quarantine policy of a government for the infectious disease is provided, the present inventors have sought to solve the problem of having to perform the infectious disease test and confirm the test results solely on an individual basis according to quarantine policy. That is, the present inventors have sought to solve the problem that when the infectious disease test is not performed on time due to an individual's deviation, the infectious disease may be transmitted to other users of one or more community groups to which the individual belongs, thereby disrupting the normal operation of the community group. As a result, the present inventors have developed the method and computing apparatus for testing the community group for the infectious disease and notifying of the test results capable of applying the infectious disease tests of the plurality of users belonging to the community group and managing the test results.

The term "infectious disease" used herein refers to a disease caused by pathogens invading a host and including proliferation of the pathogens. The pathogens include viruses, bacteria, fungi, and parasites.

The term "test result" used herein includes a positive or negative reading result analyzed through the infectious disease test. In addition, the "test result" may include cycle threshold (Ct) data of a molecular diagnostics test, infectivity of the infectious disease, severity, and treatment information based on a positive result. The treatment information may include information on the medical institution for treatment. In addition, the "test result" may further include a preventive measure for the infectious disease, vaccine information, or the like.

The term "infectious disease statistical information" used herein includes statistical data collected and analyzed from the results of each infectious disease test for the plurality of users within the community group. For example, the statistical data may include information such as the number of confirmed infections, the number of negative cases, a positive rate or negative rate, a positive rate by age, a positive rate by gender, a positive rate by region, a change in the positive rate, a change in the number of people tested, and a positive rate by the type of infectious disease, in the community group for the infectious disease test.

In addition, the "infectious disease statistical information" may include the following information by using the test results of the infectious disease test conducted periodically.
i) The infectious disease statistical information may include information on the change in the positive rate of the infectious disease test over time in the community group. The information on the change in the positive rate is information generated by displaying an extent to which a ratio of users determined to be positive among the plurality of users in a community group changes over time in a table or graph.
ii) The infectious disease statistical information may include first positive rate prediction information predicted when performing the infectious disease test on a target community group in a future using the information on the change in the positive rate. The first positive rate prediction information is information generated in the form of a table or graph, or the like, predicting the results of the next infectious disease test using the "extent to which the ratio of users determined to be positive among the plurality of users in the community group changes over time" described above.
iii) The infectious disease statistical information may include second positive rate prediction information predicted using a cycle threshold (Ct) value according to the tests of the plurality of users in the infectious disease test over time of the community group. The second positive rate prediction information is information that predicts the spread, maintenance, or decrease of the infectious disease using the change in the Ct value of each user and generates predicted values for these in the form of a table or graph.

The term "health manager" used herein refers to a user selected or designated within a community group to manage the community group. In addition, the term "health manager" refers to one or more users or an organization composed of one or more users who perform tasks for health management within the community group. For example, in a company, an educational institution, a nursing home, or the like, the health manager refers to a user or organization that performs professional health and safety-related tasks. Depending on the size of the community group, a professional health manager may be included, but depending on the form of various community groups, the user or organization that performs these health manager tasks as an assistant may be a human resources, general affairs, manager, or representative of the community group. In other words, the health manager is at least one user belonging to the community group, and may handle personal information on at least two or more users for the generation of the community group. In addition, the health manager may receive the results of the infectious disease test for the community group from a computing apparatus and perform tasks of storage and management. In addition, the health manager may confirm information on whether the plurality of users in the community group have applied for the infectious disease test and have undergone an infectious disease test.

The term "administrative district" as used herein refers to the administrative district where the community group conducting the infectious disease test is located. The administrative district in this case may be the administrative district of the region where the community group is physically located. In addition, the administrative district may be the administrative district distinguished by a government agency that manages the community group.

The administrative district may be set up in a variety of ways, from the smallest administrative district to the national administrative district. For example, in the case of South Korea, the administrative district may be set up as dong, gu, si, and do.

The term "infectious disease comparison information" used herein refers to information generated by comparing the infection index generated using the test results for the target community group with the infection index of the administrative district to which the target community group belongs.

In addition, the "infectious disease comparison information" is information generated by comparing the infection index of the target community group and the infection index of another community group in the same field as that of the target community group with each other. For example, the fields may be divided into an educational institution, a childcare institution, a general office company, a factory, a nursing home, and a shopping mall.

In addition, "infectious disease comparison information" is information generated by comparing the infection index of the target community group and the infection index of another community group having the same or similar size as that of the target community group with each other, or the infection index of the target community group and the infection index of another community group having the same or similar size and shape as those of the target community group with each other. For example, the size or shape may be selected by considering the number of people in the community group, the area of the place of the community group, the age of the users, the gender of the users, or the location of the community group.

In other words, the "infectious disease comparison information" may provide information capable of determining the level of the infectious disease management of the target community group by comparing the infection index of the target community group with various infection indexes.

The term "sampling kit" used herein refers to a tool for collecting samples for the infectious disease test. Depending on the type of infectious disease to be tested, the samples to be collected are different, and the corresponding sampling kit may be used to collect the samples. For example, in the case of respiratory infectious diseases, especially COVID-19, a tool capable of performing nasopharyngeal swab or oropharyngeal swab as an upper respiratory tract sample for PCR diagnosis is included in the sampling kit. In addition, a universal transport medium for storing the sample may also be included in the sampling kit.

These sampling kits may be delivered to the location of each community group or each of the plurality of users so that the plurality of users within the community group collect samples themselves.

Unless otherwise stated, herein, information, replies, alarms, and the like provided to each other between the infectious disease test management server, the community group, the health manager, the plurality of users, the medical institution, and the test institution are provided through a communication network using terminals among each other. The terminals used by the infectious disease test management server, the community group, the health manager, the plurality of users, the medical institution, and the test institution may include all devices capable of transmitting and receiving information through a communication network, such as PCs, mobile terminals, and wearable devices. For example, the infectious disease test management server, the community group, the health manager, the plurality of users, the medical institution, and the test institutions may use PCs. In addition, the community group, the health manager, the plurality of users, the medical institution, and the test institution may use mobile terminals.

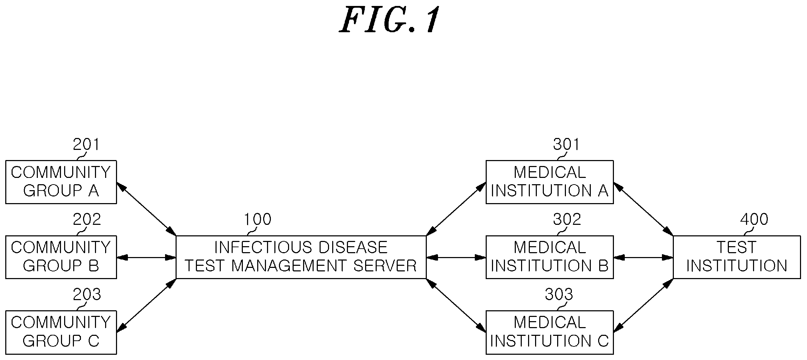

FIG. 1 is a configuration diagram illustrating a computing apparatus for testing a community group for an infectious disease and notifying the test results according to the present invention. As illustrated in FIG. 1, a computing apparatus (hereinafter referred to as an "infectious disease test management server") 100 is connected to one or more community groups 201, 202, and 203 via a communication network. In addition, the infectious disease test management server 100 is connected to one or more medical institutions 301, 302, and 303 via a communication network. In addition, the medical institutions 301, 302, and 303 are connected to a test institution 400 via a communication network for analysis of samples collected from users performing the infectious disease test.

One or more infectious disease test management server 100 may be provided. The infectious disease test management server 100 may generate one or more community groups 201, 202, and 203 using personal information on a plurality of users. The personal information on the plurality of users generating the community group may be received from each user. Alternatively, the personal information may be received from a health manager for the infectious disease management of the community group.

The infectious disease test management server 100 may provide management for the plurality of infectious diseases to the generated community groups 201, 202, and 203. For example, the infectious diseases may provide management for at least one infectious disease of COVID-19, influenza, 2022 monkeypox, sexually transmitted infections, human papillomavirus infections, respiratory infections, gastrointestinal tract infections, tuberculosis, drug resistance, or meningitis, and the type of infectious disease is not limited thereto.

A plurality of infectious diseases may be tested using various testing methods, but, preferably, a molecular diagnostics test may be used. Additionally, the infectious disease test may be performed using an antigen test, an antibody test, a culture test, or the like.

The infectious disease test management server 100 may recognize each user by using the personal information on the plurality of users included in each of the community groups 201, 202, and 203. In addition, at least one user may be included in the plurality of community groups among the community groups 201, 202, and 203. The infectious disease test management server 100 may identify the plurality of users included in the community groups 201, 202, and 203 by using the personal information on each user.

When generating each of the community groups 201, 202, and 203, the infectious disease test management server 100 may request consent for the use of personal information from the plurality of users included in each community group. The plurality of users may consent to the infectious disease test management server 100 using personal information through a corresponding reply.

In addition, the infectious disease test management server 100 may request consent for the use of personal information on the plurality of users from the health manager representing each of the community groups 201, 202, and 203. Accordingly, the health manager of the corresponding community group may request consent for the use of the personal information from the plurality of users and provide the corresponding response to the infectious disease test management server 100, thereby allowing the infectious disease test management server 100 to consent to the use of the personal information.

The infectious disease test management server 100 may provide an infectious disease test item to the community groups 201, 202, and 203 so that the community groups 201, 202, and 203 perform the infectious disease test. The infectious disease test item may include one or more infectious diseases as described previously. The infectious disease test management server 100 may receive the infectious diseases selected by the community groups 201, 202, and 203 and provide the test information for the corresponding infectious disease test.

The selection of the infectious disease by the community groups 201, 202, and 203 is preferably made by a health manager. Optionally, the selection may be made by one of the plurality of users representing the health manager.

The test information provided by the infectious disease test management server 100 includes basic information describing the infectious disease of the test subject. In addition, the test information includes test reception request information for each individual infectious disease test. The request for an infectious disease test for the community group is performed by the health manager of the community group, and the infectious disease test management server 100 may transmit test reception request information that guides the plurality of users to perform an infectious disease test.

The infectious disease test management server 100 may provide at least one selected from the group consisting of sampling method information, sampling available time information, sampling place information, test schedule information, questionnaire or medical institution reservation information, or the like to the plurality of users in response to an infectious disease test.

The sampling method information is information on the method for collecting samples from the plurality of users for the infectious disease test. For the infectious disease test, the samples should be collected from each user, and the following methods are commonly used to collect the sample.
i) The plurality of users visit the medical institution and the samples are collected.
ii) A medical professional (sample collector) of a medical institution visits a location where the plurality of users are located and the samples are collected.
iii) The plurality of users collect samples themselves (self-sampling).

The case of i) may be divided into a method in which the plurality of users visit the medical institution designated by the community group and the samples are collected and a method in which each of the plurality of users visits the medical institution selected by the user and the sample is collected.

The case of ii) may be divided into a method in which the medical professional of a medical institution visits the community group to collect the samples and a method in which the medical professional of the medical institution visits the places at which the plurality of users are located to collect the samples.

The self-sampling method is a method in which the sampling kit is provided to each of the plurality of users, and the plurality of users collect samples themselves. The collected samples may be provided to the medical institutions or test institutions designated by the plurality of users.

The sampling kits may be delivered from the medical institution or the test institution. The sample kit may be delivered to the community group or to the place designated by the user.

Sampling kits may be provided to the user visiting the medical institution or test institution.

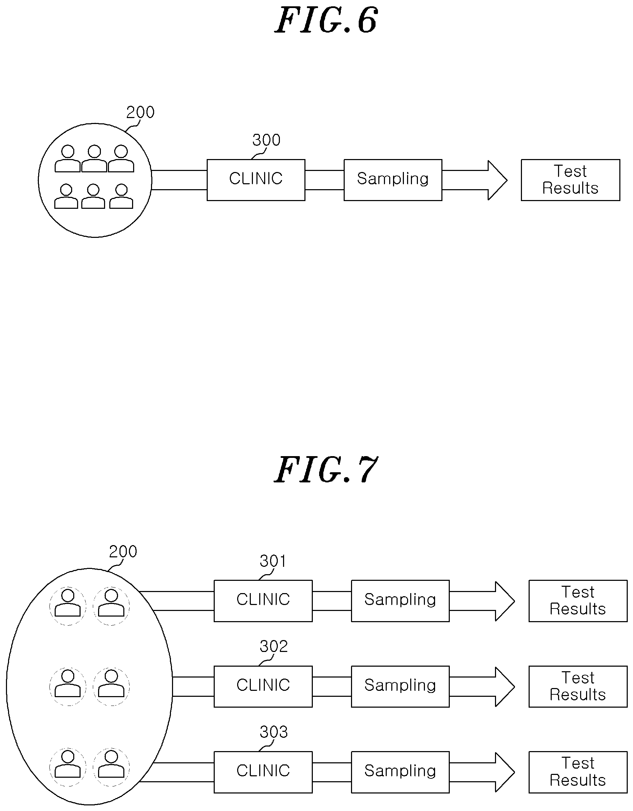

Descriptions of the sampling method information may be described in detail through FIGS. 6 to 10.

FIG. 6 is a first exemplary diagram illustrating a method for collecting samples for the infectious disease test for the community group according to the present invention. As illustrated in FIG. 1, the plurality of users of the community group 200 may visit the medical institution 300 and the samples may be collected (sampling) by the medical professional of the medical institution 300.

In one implementation example of the present invention, the medical institution 300 may be a medical institution designated by the community group 200.

In another implementation example of the present invention, the medical institution 300 may be a medical institution designated by the infectious disease test management server 100.

FIG. 7 is a second exemplary diagram illustrating the method for collecting samples for the infectious disease test for the community group according to the present invention. As illustrated in FIG. 7, the plurality of users of the community group 200 may visit the plurality of medical institutions 301, 302, and 303 and the samples may be collected by the medical professionals of the medical institutions 301, 302, and 303.

In one implementation example of the present invention, the samples of the plurality of users may be collected by the medical institution selected by each user among the plurality of medical institutions 301, 302, and 303.

In another implementation example of the present invention, the sample of each user among the plurality of users may be collected by the medical institution designated by the community group 200 among the plurality of medical institutions 301, 302, and 303.

The plurality of medical institutions 301, 302, and 303 may provide the test results for users who have collected samples from among the plurality of users to the infectious disease test management server 100.

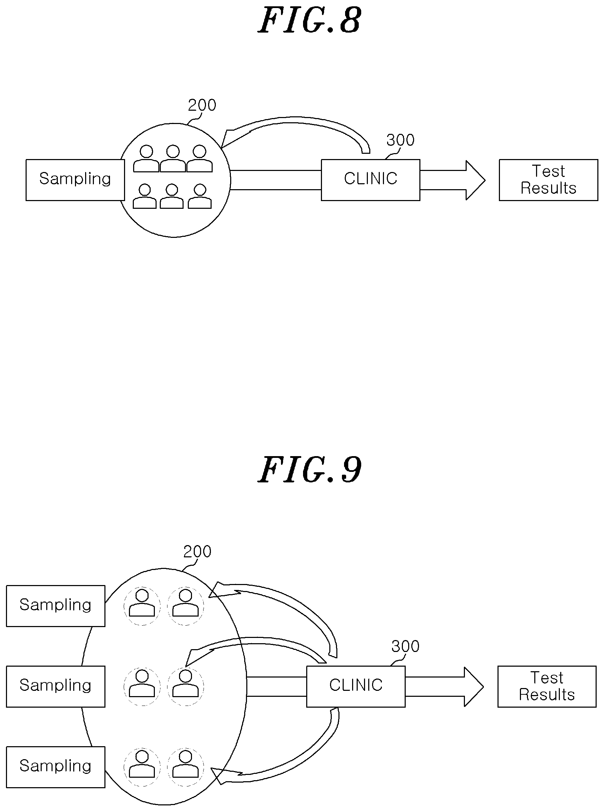

FIG. 8 is a third exemplary diagram illustrating the method for collecting the samples for the infectious disease test for the community group according to the present invention. As illustrated in FIG. 8, the samples of the plurality of users of the community group 200 may be collected by the visit from the medical staff of the medical institution 300. At this time, the place visited by the medical staff may be only one community group 200.

That is, the medical staff may visit the community group 200 during a sampling available time and collect the sample from each of the plurality of users. For example, since the plurality of users of companies, shopping malls, educational institutions, or the like generally live within the community group 200, the samples may be collected from the same place upon the visit of the medical staff.

FIG. 9 is a fourth exemplary diagram illustrating a method for collecting the samples for the infectious disease test for the community group according to the present invention. As illustrated in FIG. 9, one medical institution 300 may collect samples from the users located in the plurality of places.

In one implementation example of the present invention, the place visited by the medical staff may be a place designated by each of a plurality of users. For example, each user may designate the community group 200, home, current location, specific place, or the like as a sampling place. The medical staff may visit the place selected by the user and collect the sample.

In another implementation example of the present invention, the place visited by the medical staff may be a plurality of places designated by the community group 200 or the medical institution 300 or the infectious disease test management server 100. The plurality of users may select one of the plurality of designated places to collect the samples. For example, when the plurality of users are drivers located outside, or the like, the plurality of users should continuously move. Therefore, when the drivers select one of the plurality of designated places, the samples of the drivers may be collected by the medical staff visiting the selected place.

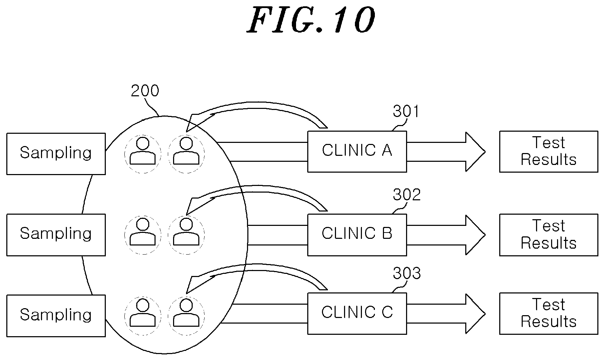

FIG. 10 is a fifth exemplary diagram illustrating the method for collecting the samples for the infectious disease test for the community group according to the present invention. As illustrated in FIG. 10, the plurality of users of the community group 200 select one of the medical institutions 301, 302, and 303. Then, the medical staff of the selected medical institution request the plurality of users to visit to the designated place.

In one implementation example of the present invention, the sample collection place visited by each medical professional of the medical institutions 301, 302, and 303 may be the place designated by each of the plurality of users.

In another implementation example of the present invention, the sample collection place visited by each medical professional of the medical institutions 301, 302, and 303 may be a location designated by the community group 200, each of the medical institutions 301, 302, and 303, or the infectious disease test management server 100.

As illustrated in FIGS. 6 to 10, the sampling place has been described through various implementation examples, but the method of selecting the sampling place is not limited thereto, and other implementation examples may be implemented through various embodiments.

The sampling available time information is the time information at which the samples or the sample of all users or each user among the plurality of users may be collected. Depending on the characteristics of each community group, the plurality of users may be located in the community group only at a specific time. Alternatively, each of the plurality of users may be located in the community group at different times. Depending on the characteristics of these community groups, the time at which the samples may be collected may be provided by the community group or the plurality of users.

When the sampling available time is determined by the community group or the health manager of the community group, the plurality of users may omit the reply regarding the sampling available time information.

When the plurality of users visiting the medical institution to collect the samples thereof is selected, the medical institution reservation information may replace the sampling available time information.

The sampling place information is information provided for the medical staff (sample collectors) of the medical institution to visit the location where the plurality of users are located. The medical staff may visit the place to collect the samples from the plurality of users of the community group. The place at this time may be a place where the community group is located, a place where the plurality of users are currently located, a place selected by the plurality of users, a place designated by the medical institution, or a place designated by the community group (health manager). The plurality of users should provide the location information for the selected place to the infectious disease test management server 100. The location information at this time may include location information using an address, location information using a GPS signal, or location information based on latitude/longitude.

When a sampling place, for example, a sample collection place, is designated, the plurality of users may go to the sample collection place and the samples thereof may be collected by the medical staff.

When the sampling place is not designated, the plurality of users may reply to the infectious disease test management server 100 with location information they have selected and the samples thereof may be collected by the medical staff visiting the selected location.

The test schedule information is information that includes a test schedule applied to the community group or each of the plurality of users. The infectious disease test for the community group is performed periodically, and the infectious disease tests for the plurality of infectious diseases may be performed. At this time, the schedule for infectious disease tests performed periodically for the community group may be established. In addition, the schedule for infectious disease tests for the plurality of infectious diseases may be established.

In one implementation example of the present invention, the schedule for each infectious disease test may be automatically generated by the infectious disease test management server 100 and provided to the community group or the plurality of users.

In another implementation example of the present invention, the schedule for each infectious disease test may be generated by the medical staff and stored on the infectious disease test management server 100.

In another implementation example of the present invention, the schedule for each infectious disease test may be generated by the community group (health manager) and provided to the infectious disease test management server 100, so that the infectious disease test management server 100 provides the schedule to the plurality of users.

The schedule for conducting the infectious disease test may be, but is not limited to, a weekly schedule, a monthly schedule, a quarterly schedule, a semi-annual schedule, an annual schedule, and/or a set periodic schedule. Accordingly, test information is provided to the plurality of users according to the corresponding test schedule.

Additionally, the test schedule may further include a notification schedule. The notification schedule is an anticipated date by which the test results of the infectious disease test scheduled for testing will be notified.

The infectious disease test may be performed for the plurality of infectious diseases, and the test schedule information may further include schedules for other infectious disease test applied to all or each of the plurality of users of the community group.

The questionnaire may display questions about the infectious disease to be tested and receive corresponding answers. The questionnaire is classified into questions that are commonly applied to all infectious disease tests and questions that are classified according to the characteristics of each infectious disease test, and is stored on the infectious disease test management server 100.

The questionnaire may be written in text format by the user in the form of text questions. The questionnaire may be written in the form of selecting one or more of the plurality of expected answers provided. The questionnaire may be written by providing the shape of the body in graphic form and allowing the user to select the location where the symptom occurred. The questionnaire may be input by the user while proceeding through the plurality of steps for each infectious disease test.

The medical institution reservation information is information provided to the plurality of users to allow each of the plurality of users to make reservations for visiting a medical institution for sample collection. Alternatively, as described above, sampling available time information and sampling place information may be included in the medical institution reservation information.

The plurality of users may make reservations at the medical institution for the sample collection by referring to the medical institution reservation information. When the sample collection place is designated as a medical institution, the sampling available time information may be included in the medical institution reservation information and replied to the infectious disease test management server 100.

The infectious disease test management server 100 may receive at least one of the reply to the test reception request information, the reply to the sampling method information, the reply to the sampling available time information, the reply to the questionnaire, and the reply to the medical institution reservation request from a plurality of users, in response to test information provided to the plurality of users.

The infectious disease test management server 100 may receive test results of the infectious disease tests performed by the plurality of users of community groups 201, 202, and 203.

In one implementation example of the present invention, the test results may be received from the medical institutions 301, 302, and 303 that collect the samples from the plurality of users.

In another implementation example of the present invention, the test results may be received from the test institution 400 that tests samples of the plurality of users.

The infectious disease test management server 100 may receive the test results for the plurality of users of community groups 201, 202, and 203 and generate an integrated test result. The integrated test result may include individual test results corresponding to the plurality of users.

Additionally, the integrated test results may include an infectious disease statistical information for the target community group using the individual test results corresponding to the plurality of users.

The integrated test result only includes the test results and an infectious disease statistical information for the plurality of users included in the target community group.

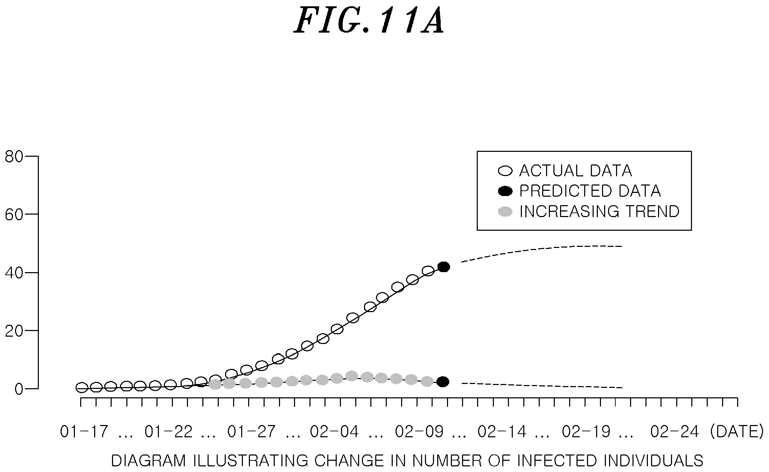

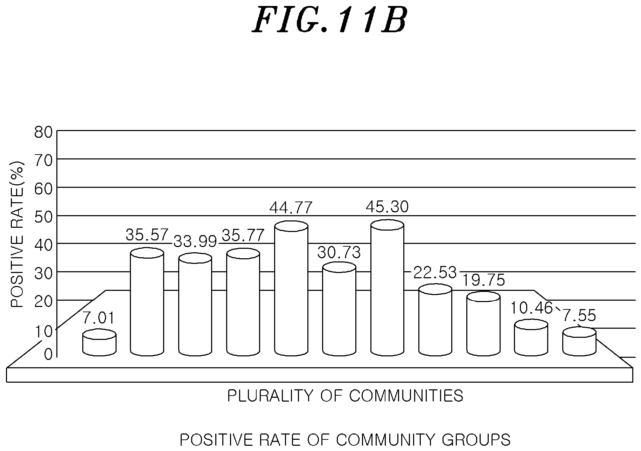

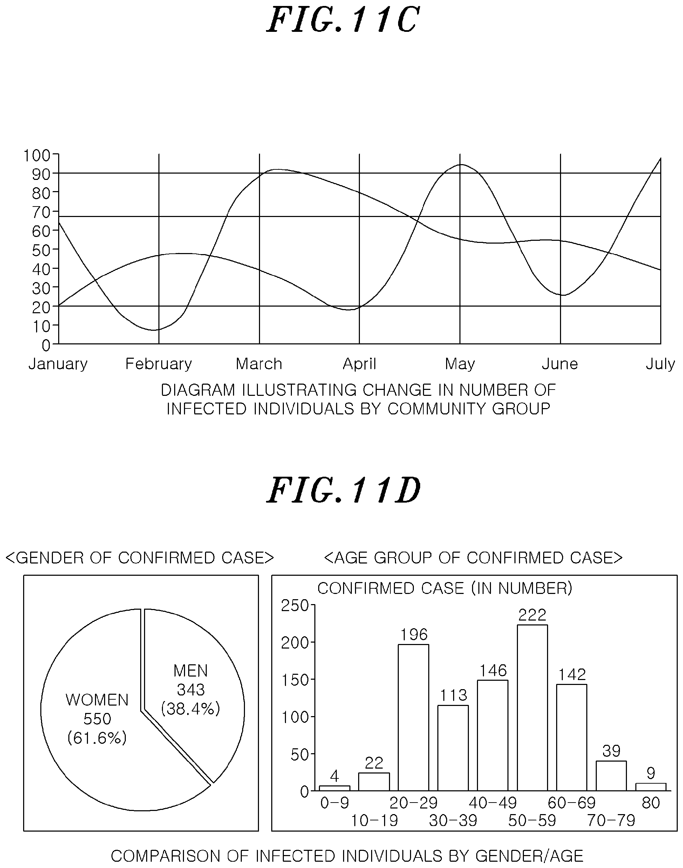

The infectious disease statistical information among the integrated test results is explained with reference to FIG. 11.

FIG. 11 is exemplary diagrams for illustrating integrated test results using the test results according to the present invention. As illustrated in FIG. 11, the infectious disease test management server 100 may generate various infectious disease statistical information using the test results for the plurality of users included in the community group.

In one implementation example of the present invention, the infectious disease statistical information may include a diagram illustrating a change in the number of infected individuals as illustrated in FIG. 11A. The infectious disease test management server 100 may perform the periodic infectious disease test on the community group and use the accumulated test results to generate the diagram illustrating the change in the number of infected individuals occurring in the community group. As illustrated in the graph, the diagram illustrating the change in the number of infected individuals may be more easily confirmed by the health manager of the community group or the plurality of users.

For example, as in FIG. 11A, the diagram illustrating the change in the number of infected individuals may generate data by the date on which the infection test is performed. In addition, the diagram illustrating the change in the number of infected individuals may include the predicted number of infected individuals and actual data (actual number of infected individuals) within the community group, and may also illustrate the increasing trend in the actual data.

In another implementation example of the present invention, the infectious disease statistical information may include a graph illustrating the positive rate of the community group, as in FIG. 11B. For example, the infectious disease test management server 100 may calculate the positive rates for the infectious disease tests for the plurality of communities and display the positive rates as in FIG. 11B. The positive rates calculated for the plurality of communities may be compared with each community.

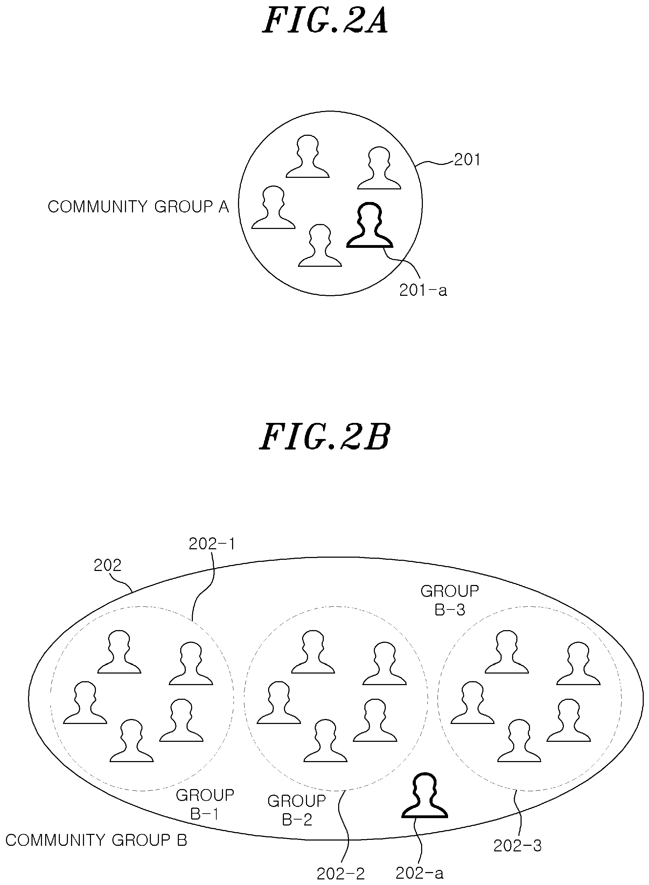

In another implementation example of the present invention, the infectious disease statistical information may include a diagram illustrating a change in the number of infected individuals according to a period by community group, as in FIG. 11C. As illustrated in FIG. 2B, the community group may include the plurality of small groups, and the infectious disease test management server 100 may generate a diagram illustrating a change in the number of infected individuals for each small group and cause the diagram illustrating the change to be included in the infectious disease statistical information.

In still another implementation example of the present invention, the infectious disease statistical information may include a comparison of infected individuals by gender/age, as in FIG. 11D. The health manager of the community group may establish infectious disease management measures through various distribution maps of infected individuals within the provided community group. For example, the confirmed cases of infectious diseases in the community group may be classified by gender. Alternatively, the confirmed cases of infectious diseases may be classified by age group. The age may be classified as 0 to 9 years old, 10 to 19 years old, 20 to 29 years old, 30 to 39 years old, 40 to 49 years old, 50 to 59 years old, 60 to 69 years old, 70 to 79 years old, 80 years old or older, or the like. The ages classified in the age group classification may be classified in various ways according to the characteristics of the users belonging to the community group.

The implementation examples and graphs included in the drawings presented in FIG. 11 is an only example of each embodiment, and various statistical information and graphs may be generated and provided to the community group.

The infectious disease test management server 100 may generate the infection index using the test results for the plurality of users. In addition, the infectious disease test management server 100 may generate the infectious disease comparison information by comparing the infection index of the community group with the infection index using the infectious disease test results for at least one of the administrative district including the community group and another community.

In one implementation example of the present invention, the infection index may include at least one of an infection rate (positive rate) for the infectious disease, a basic reproduction number, a local safety index, and a contagion index.

In another implementation example of the present invention, the infection index may further include other infection indexes other than those described above.

The infection rate (positive rate) or incidence rate refers to the proportion of patients who develop the infectious disease when the generated infectious disease is limited to a certain region and period.

The basic reproduction number, or basic infection reproduction number, represents the average number of secondary infections that a primary infection case may cause when the first infected individual occurs in a population without any infected individuals.

The local safety index is a rating that quantifies the safety level of each local government by utilizing various statistics on safety. There are various calculation fields, but the present invention may calculate the local safety index for infectious diseases.

The contagion index is the probability that the pathogens will cause an infection in a host, and is usually expressed as a percentage of the number of patients divided by the number of infected individuals.

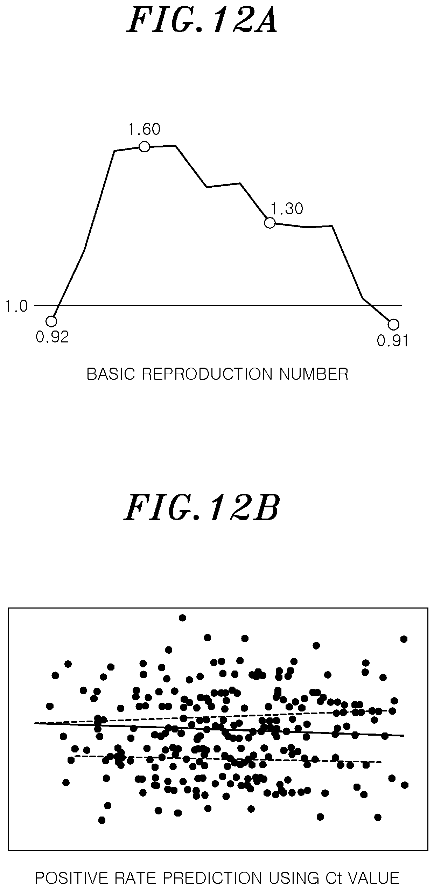

The infection index is explained with reference to FIG. 12.

FIG. 12 is an exemplary diagram illustrating the infection index using the test results according to the present invention.

In one implementation example of the present invention, the infection index may include the basic reproduction number, as illustrated in FIG. 12A.

In another implementation example of the present invention, the infection index may include positive rate prediction using the Ct value, as illustrated in FIG. 12B.

Although not illustrated in the drawing, in one implementation example of the present invention, the infectious disease test management server 100 may generate infectious disease comparison information obtained by comparing various statistical information and infection indexes for the infectious disease test for the community group with the statistical information and infection indexes of other community groups.

In another implementation example of the present invention, the infectious disease test management server 100 may generate infectious disease comparison information obtained by comparing various statistical information and infection indexes on the infectious disease tests of the community group with the statistical information and infection indexes of the administrative district including the community group.

The infectious disease test management server 100 may provide the generated infectious disease comparison information to the health manager of the community group. The health manager may compare the level of the infectious disease management of the community group with the administrative district through various infectious disease comparison information and compare the infectious disease management level with other community groups.

In one implementation example of the present invention, the other community group may be a community group of the same or similar series. For example, when the community group is a religious facility, the other community group to be compared may also be selected as a religious facility.

In another implementation example of the present invention, the other community group may be a community group of the same or similar size. For example, when the community group is an educational institution with about 200 people, the other compared community group may also be a community group of about 200 people. In this case, the series of the community groups may be the same or different.

In still another implementation example of the present invention, the other community group may be a community group designated by a health manager or the infectious disease test management server 100. The infectious disease test management server 100 may generate and provide infectious disease comparison information compared to a specific community group designated by the health manager.

When the negative test results are received for the user who has been determined to be positive in the infectious disease test previously performed among the plurality of users in the community group, the infectious disease test management server 100 generates return time point information on a time point when the user who have been determined to be negative returns to the community group. For example, the return time point information generated when the user who has been determined to be positive for the infectious disease test is determined to be negative is preferably generated by a rule such as i) when the user is determined to be negative in the result of a first infectious disease test after the user is determined to be positive, or ii) when the user is determined to be negative again in the result of a second infectious disease test within 2 days or 3 days after the user is determined to be negative in the result of the first infectious disease test after the user is determined to be positive. The return time point information is included in at least one of the test result and the integrated test result of the corresponding user.

The return time point information may be generated using information such as the age, gender, job responsibilities within the community group, Ct value of the infectious disease test for the negative result, or the period of the positive result of the user.

In one implementation example of the present invention, the return time point information may be automatically generated by the infectious disease test management server 100 and provided to the corresponding user.

In another implementation example of the present invention, the return time point information may be generated by the medical institutions 301, 302, and 303 and provided to the corresponding user through the infectious disease test management server 100.

In still another implementation example of the present invention, the return time point information may be generated by an administrative agency (not illustrated) and provided to the corresponding user through the infectious disease test management server 100.

In still another implementation example of the present invention, the return time point information may be received by the infectious disease test management server 100 from the health manager of the community group and provided to the corresponding user.

The infectious disease test management server 100 may provide quarantine guidelines generated for the community group to the corresponding user among the plurality of users.

The quarantine guideline includes an infectious disease-related content that should be carried out before and after the infection with an infectious disease. In other words, the quarantine guidelines may include information on daily life, vaccination, treatment, eating habits, use of hygiene tools, isolation, infectious disease test items by symptoms, behavioral rules by positive/negative, or the like.

In one implementation example of the present invention, the quarantine guidelines may be generated by the infectious disease test management server 100 and provided to the corresponding user among the plurality of users.

In another implementation example of the present invention, the quarantine guidelines may be generated by the administrative agency and provided to the corresponding user through the infectious disease test management server 100.

In still another implementation example of the present invention, the quarantine guidelines may be received by the infectious disease test management server 100 from the health manager of the community group and provided to the corresponding user.

The infectious disease test management server 100 generates a certificate for infectious disease management and/or infectious disease safety for the corresponding community group when the test result and/or integrated test result of the infectious disease test performed on the community group is equal to or more than a predetermined threshold. The generated certificate may be provided to the community group (health manager).

For example, among users belonging to the entire community group, a case where the test results of the infectious disease test are negative at 100%, 95% or more, 85% or more, or 80% or more may be considered to be equal to or more than a predetermined threshold.

The certificate may be used as a criterion to determine whether a community group is at a safe quarantine level based on the infectious disease tests of the plurality of users.

For example,
i) In the case of educational institution, when a large number of educators and trainees are located within the community group, but the number of infectious disease patients is equal to or more than a certain level, the infectious disease may become uncontrollable. However, when the number of infectious disease patients is equal to or less than a certain level, the community group may be controlled from the infectious disease, and the large number of educators and trainees, or the like may be safe from the infectious disease.
ii) In the case of a shopping mall with a plurality of stores, many employees are working and many customers are visiting. In this case, when the number of infected employees among many employees is equal to or more than a certain level, the risk of infection for other employees and customers located in the shopping mall may increase. However, when it is possible to control the infectious disease by sufficiently implementing quarantine measures based on positive test results for some employees, the shopping mall may operate normally and attract customers to visit the shopping mall where the customers are safe from the infectious disease.

As mentioned above, various community groups are spaces where the plurality of users live or where customers stay. In particular, the employees of the community groups that face customers should periodically undergo the infectious disease test to ensure the safety of customers. Moreover, by proving to customers that the community group is safe through periodically conducted infectious disease test, the customers will continue to visit the community group.

Proving to customers that the community group is safe may be done through the certificate generated by the infectious disease test management server 100. That is, the community group that receives the certificate for the infectious disease test that is performed periodically may prove that the community group is a community group that is safe from the infectious disease by presenting/providing the certificate to the customers. Accordingly, the infectious disease test management server 100 may generate the certificate according to the infectious disease test for the community group and provide the certificate to the community group or health manager.

Additionally, the community group may provide the certificate so that the plurality of users and/or customers may confirm the certificate. However, the infectious disease test management server 100 may display the certificate on a digital map provided by the infectious disease test management server 100 so that the plurality of users and/or customers may more easily verify the generated certificate. In this case, it is preferable that the certificate displayed on the digital map provided by the infectious disease test management server 100 is displayed at the place where the community group is located. Accordingly, certificates for the plurality of community groups may be displayed together on the digital map provided by the infectious disease test management server 100. The plurality of users and/or customers may confirm the certificates of the plurality of community groups displayed on the digital map and confirm the level of the infectious disease management of the community group that they wish to visit.

In addition, the certificate displayed on the digital map may be displayed on a digital map provided by an external computing apparatus (not illustrated). For example, the external computing apparatus is a computing apparatus that provides a map service through an application, website, or the like so that customers may easily access the external computing apparatus through a mobile terminal. To this end, the infectious disease test management server 100 may provide the certificate and certificate-related information (including location information on a community group) to the external computing apparatus. The external computing apparatus may display the received certificate and certificate-related information on the map provided.

The certificate-related information may include location information on the community group where the certificate should be displayed. The location information may use location data such as an address, a GPS signal, a latitude/longitude, or the like. The certificate-related information may also include the infectious disease information.

The community groups 201, 202, and 203 are groups that include the plurality of users. The community groups 201, 202, and 203 include at least one health manager for the infectious disease test. Descriptions of the community groups are provided with reference to FIG. 2.

FIG. 2 is an exemplary diagram illustrating various forms of the community groups according to the present invention. As illustrated in FIG. 2, the community groups may be divided into the forms of a community group A 201 and a community group B 202.

The community group A 201 referring to FIG. 2A includes a plurality of users, and at least one of the plurality of users is a health manager 201-a.

The community group B 202 referring to FIG. 2B, includes a plurality of small groups 202-1, 202-2, and 202-3, and includes at least one health manager 202-a who manages the infectious disease test for each of the plurality of small groups 202-1, 202-2, and 202-3.

In one implementation example of the present invention, the health manager 202-a may not be included in the plurality of small groups 202-1, 202-2, and 202-3 and may manage each small group as a whole.

In another implementation example of the present invention, the health manager may be included in any one small group of the plurality of small groups 202-1, 202-2, and 202-3 and may manage each small group as a whole.

In another implementation example of the present invention, the health manager may be included in each of a plurality of small groups 202-1, 202-2, and 202-3 and may manage each small group.

The community groups 201, 202, and 203 referring to FIG. 1 include terminals capable of communicating with the infectious disease test management server 100.

In one implementation example of the present invention, the terminal is a computing apparatus capable of communication, such as a PC and/or a mobile terminal (personal portable communication device). The terminal belongs to the community groups 201, 202, and 203 and may be operated by each health manager.

In another implementation example of the present invention, the terminal is a communication-capable computing apparatus, such as a PC and/or mobile terminal, owned by each health manager of the community groups 201, 202, and 203. The terminal may be operated by the health manager.

The health manager may provide personal information on the plurality of users for the generation of the community group provided through the terminal to the infectious disease test management server 100. The health manager may receive the test results of the infectious disease tests of the plurality of users through the terminal. The health manager may receive the integrated test result from the infectious disease test management server 100 through the terminal. The health manager may receive the certificate for infectious disease management from the infectious disease test management server 100 through the terminal.

The community groups 201, 202, and 203 are organizations that include the plurality of users. For example, the community groups are organizations such as an educational institution, a childcare institution, a postnatal care center, a company, a car sharing service, a transportation, a religious facility, a restaurant, a travel agency, a nursing home, a performance facility, a sports facility, and a shopping mall.

Example community groups include users who belong to the group, and may include customers if necessary.

For example, when the community group is a nursing home, the staff working at the nursing home and the patient admitted to the nursing home may be included in the plurality of users. When the community group is a performance facility, the staff working at the performance facility and the performers performing the performance may be included in the plurality of users. Meanwhile, when the audience watching the performance is specified, the audience may also be included in the plurality of users or customers. When the community group is a travel agency, the travel agency employees and the travelers who purchased the travel product may be included in the plurality of users.

In the event of a socially spread infectious disease, customers want to visit facilities that are safe from infectious diseases. Therefore, the community groups that customers visit need to inform the customers that the community groups are safe from infectious diseases. To this end, the infectious disease test management server 100 may manage the infectious disease tests for community groups and generate the certificate that authenticates a safe community group.

Meanwhile, in the case of a community group that includes customers among the plurality of users included in the community groups 201, 202, and 203, the community group may be classified into two small groups. A first small group is a plurality of users including employees belonging to the community group, and a second small group is a plurality of users including customers who visit temporarily. Therefore, it is preferable that the second small group be generated temporarily and deleted after the infectious disease test.

The medical institutions 301, 302, and 303 may include one or more medical staffs to perform the infectious disease test on the plurality of users of the community group. Preferably, the medical institutions 301, 302, and 303 may collect samples for the infectious disease test from the plurality of users.

In one implementation example of the present invention, there may be the plurality of medical institutions 301, 302, and 303 illustrated in FIG. 1.

In another implementation example of the present invention, the medical institution may be one.

The medical institutions 301, 302, and 303 may collect the samples through the medical staff when the plurality of users visit the medical institution.

In one implementation example of the present invention, the plurality of users may provide reservation information to the infectious disease test management server 100 to visit the medical institution.

In another implementation example of the present invention, the plurality of users may check in on-site at the medical institution without a separate reservation.

The medical institutions 301, 302, and 303 may be connected to the infectious disease test management server 100 and provide the test results. In addition, medical institution schedule information provided through the infectious disease test management server 100 may be received to collect the samples from the plurality of users.

The test institution 400 actually tests the samples of the plurality of users collected from medical institutions 301, 302, and 303. The test institution 400 may test the samples of the plurality of users to determine whether the plurality of users are positive or negative for the infectious disease, and provide the determined test results to the medical institutions 301, 302, and 303.

In one implementation example of the present invention, one test institution 400 is provided in FIG. 1.

In another implementation example of the present invention, a plurality of test institutions may be provided.

In another implementation example of the present invention, the test institution may be provided with a one-to-one match with the medical institution.

In another implementation example of the present invention, the test institution 400 may be the medical institution. That is, at least one medical institution of the medical institutions 301, 302, and 303 may be equipped with testing personnel and testing equipment and may perform actual testing for infectious diseases. In this case, the infectious disease test management server 100 may receive the test results generated by the medical institution.

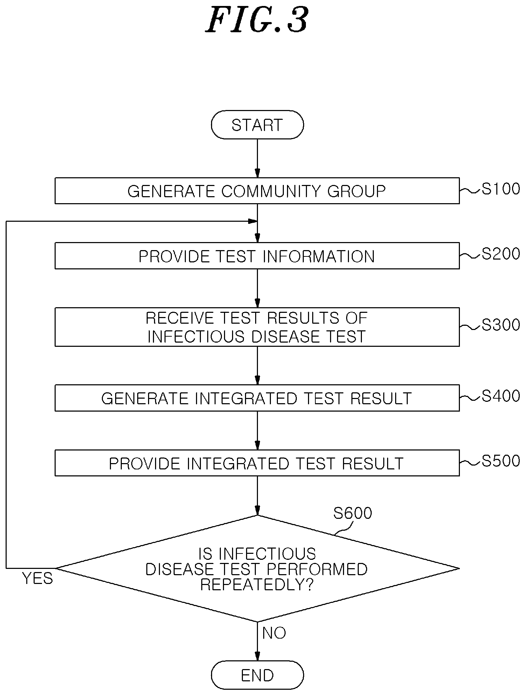

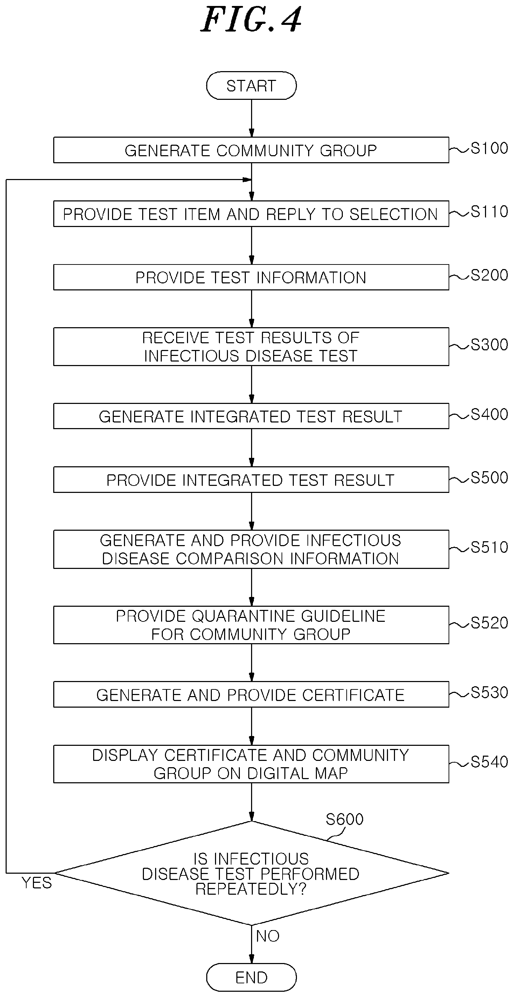

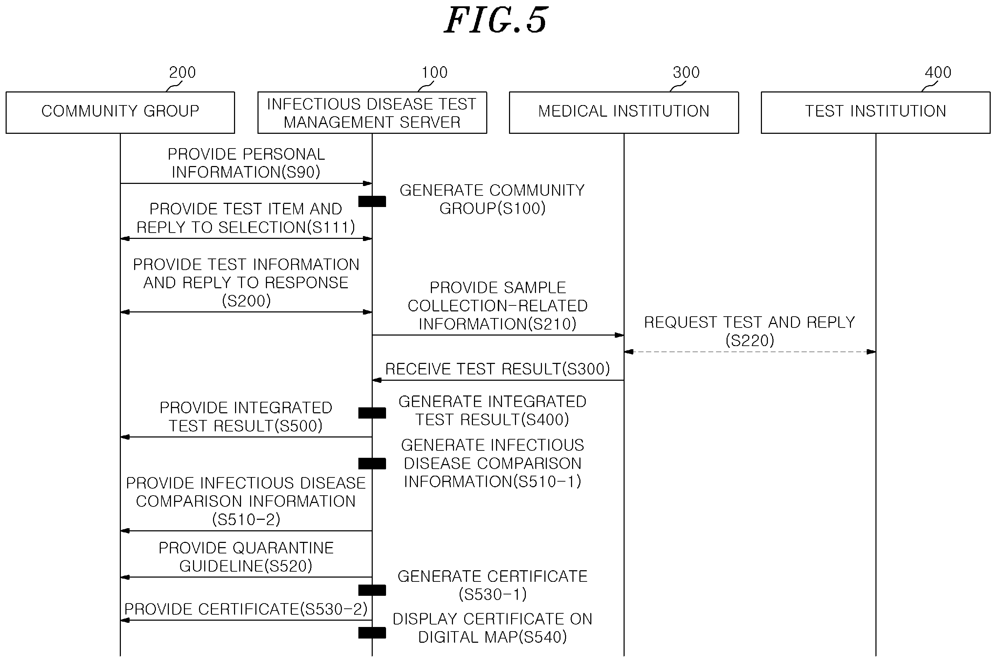

FIG. 3 and FIG. 4 are sequence diagrams illustrating the method for testing the community group for the infectious disease and notifying the results of the test according to the present invention. In addition, FIG. 5 is a flowchart illustrating the method for testing the community group for the infectious disease and notifying the results of the test according to the present invention.

As illustrated in FIGS. 3 to 5, the infectious disease test management server 100 may generate a community group including the plurality of users in order to perform infectious disease test application and test result notification for the community group (S100).

The community groups generated in Step S100 are generated using personal information on the plurality of users.

In one implementation example of the present invention, the personal information on a plurality of users may be directly provided by each of the plurality of users to the infectious disease test management server 100.

In another implementation example of the present invention, the personal information on the plurality of users may be provided by a health manager of the community group to the infectious disease test management server 100.

In addition, in one implementation example of the present invention, the infectious disease test management server 100 may request consent from the plurality of users to provide the test results of infectious disease test to the community group. The infectious disease test management server 100 may receive the reply to the consent request from the plurality of users.

In another implementation example of the present invention, the infectious disease test management server 100 may request consent from a health manager to provide the test results of the infectious disease test to the community group. The infectious disease test management server 100 may receive the reply to the consent request generated by the plurality of users from the health manager.

The infectious disease test management server 100 may generate the plurality of community groups, and at least one user among the plurality of users may be included in two or more community groups.

The community group generated by the infectious disease test management server 100 may be at least one selected from the group consisting of an educational institution, a childcare institution, a postnatal care center, a company, a car sharing service, a transportation, a religious facility, a restaurant, a travel agency, a nursing home, a performance facility, a sports facility, and a shopping mall.

The infectious disease test management server 100 may provide the test items for the infectious disease test to the health manager of the generated community group and reply to the test item selected by the health manager (S110).

In Step S110, the infectious disease test management server 100 may provide a plurality of infectious disease test items to at least one of the health manager and the plurality of users for the selection of the infectious disease test to be performed in the community group. The infectious disease test management server 100 may receive a test item selected by at least one of the health manager and the plurality of users among the provided infectious disease test items.

In one implementation example of the present invention, the plurality of infectious disease test items provided in Step S110 may include at least one selected from the group consisting of COVID-19, influenza, monkeypox 2022 monkeypox, sexually transmitted infections, human papillomavirus infections, respiratory infections, gastrointestinal tract infections, tuberculosis, drug resistance, or meningitis.

In another implementation example of the present invention, the plurality of infectious disease test items provided in Step S110 may further include at least one other infectious disease including the items.

The infectious disease test management server 100 may provide the test information on the infectious disease test to the plurality of users included in a generated community group (S200).

The test information provided in Step S200 may include at least one selected from the group consisting of test reception request information, sampling method information, sampling available time information, sampling place information, test schedule information, questionnaire, and/or medical institution reservation request information for the infectious disease test.

In this case, the sampling method information may be about a method for collecting (sampling) samples from the plurality of users for the infectious disease test, and may be implemented as follows.
i) The plurality of users visit the medical institution.
ii) The sample collector visits the location where the plurality of users are located.
iii) The plurality of users directly collect the samples.

The sampling method information may include information that allows the plurality of users to select one of the above methods or information that is provided to the plurality of users about a pre-selected sampling method.

Among the sampling method information,
i) When the plurality of users visiting the medical institution is selected, the infectious disease test management server 100 receives location information from each of the plurality of users and provides information on at least one medical institution adjacent to each of the received location information to the plurality of users.
ii) When the sample collector visiting the location where the plurality of users are located is selected, the infectious disease test management server 100 receives location information from each of the plurality of users and provides each of the received location information to the medical institution or sample collector.
iii) When the plurality of users directly collecting samples is selected, the infectious disease test management server 100 may receive location information from each of the plurality of users and set the received location information as the delivery location of the sampling kit. The infectious disease test management server 100 may provide the location information or delivery location to the medical institution or test institution that holds the sampling kit so that the sampling kit may be delivered to the set delivery location.

The test schedule information is a schedule for performing the infectious disease tests that are applied to all or each user of a community group. The schedule for performing the infectious disease tests may include at least one of a weekly schedule, a monthly schedule, a quarterly schedule, a semi-annual schedule, an annual schedule, and a set periodic schedule.

For example,
i) When the number of confirmed cases is equal to or more than or equal to or less than a threshold value based on an infectious disease test, the periodic schedule may be a schedule at which the infectious disease test is conducted every 1, 3, 5, or 10 days.
ii) When the infection index of the administrative district including the community group is equal to or more than or equal to or less than the threshold value, the periodic schedule may be a schedule at which the infectious disease test is conducted every 1, 3, 5, or 10 days.
iii) When a confirmed case occurs within a community group, the periodic schedule may be a schedule at which the infectious disease test is conducted every 1, 3, 5, or 10 days.

The test information may be provided to the plurality of users according to the test schedule information described above.

In one implementation example of the present invention, the test schedule information may further include the notification schedule for test results of the infectious disease test.

In another implementation example of the present invention, the test schedule information may further include schedules for other infectious disease tests applied to the entire community group or to each of a plurality of users.

The infectious disease test management server 100 may receive at least one selected from the group consisting of the reply to the test reception request information, the reply to the sampling method information, the reply to the sampling available time information, the reply to the questionnaire, or the reply to the medical institution reservation request from a plurality of users, in response to the test information provided to the community group or the plurality of users.

The infectious disease test management server 100 may receive the test results of the infectious disease test conducted on the plurality of users S300. The test results may be received from the medical institution or the test institution that performed the infectious disease test.

In one implementation example of the present invention, the medical institution collects the samples for the infectious disease test, and the collected samples are provided to the test institution, so that the test institution may perform the actual test for the infectious disease. In this case, the test results may be directly provided from the test institution to the infectious disease test management server 100. Alternatively, the test institution may provide the test results to the medical institution, and the infectious disease test management server 100 may receive the test results through the medical institution.

In another implementation example of the present invention, the medical institution may be the test institution. That is, the medical institution may collect samples from the plurality of users and perform actual infectious disease tests using testing personnel and testing equipment provided by the medical institution. In this case, the infectious disease test management server 100 may receive the test results generated by the medical institution.

The infectious disease test conducted on the plurality of users may use molecular diagnostics tests.

The infectious disease test management server 100 may use the test results received in Step S300 to generate the integrated test result for the community group (S400).

The infectious disease test management server 100 may provide the integrated test result generated in Step S400 to the health manager of the community group (S500). The integrated test result includes an infectious disease statistical information for the community group generated using each test result corresponding to the plurality of users.

Using a previously conducted infectious disease test including the above infectious disease test,
i) the infectious disease statistical information may include information on the change in the positive rate of the infectious disease test over time,
ii) the infectious disease statistical information may include information on predicting the positive rate using the change in the positive rate, and
iii) the infectious disease statistical information may include information on predicting the positive rate using a cycle threshold (Ct) value of the infectious disease test over time.

The infectious disease test management server 100 may include the test result of each of the plurality of users in the integrated test result.

The infectious disease test management server 100 generate the infectious disease comparison information obtained by comparing the infection index generated using the test results received in Step S300 with the infection index generated using the infectious disease test results for at least one of the administrative district and another community including the community group (S510-1), and provides the generated infectious disease comparison information to the health manager of the community group (S510-2) (S510).

The infection index generated by the infectious disease test management server 100 may include at least one of the infection rate (positive rate), basic reproduction number, local safety index, and contagion index for the tested infectious disease.

The infectious disease test management server 100 may generate additional return time point information after Step S300. The return time point information is information on the time when a user who is determined to be negative returns to the community group when a negative result for an infectious disease test is received after a positive result among the plurality of users included in the community group. The return time point information may be provided to be included in at least one of the test result of the user who is determined to be negative and the integrated test result.

In one implementation example of the present invention, the return time point information may be included in the test results provided to a user who has been determined to be negative.

In another implementation example of the present invention, the return time point information may be included in the integrated test result provided to health managers of the community group.

The infectious disease test management server 100 may provide the quarantine guideline stored for the community group to the corresponding user among the plurality of users (S520).

When the test results and/or integrated test results for the plurality of users belonging to a community group are equal or more than a threshold value, the infectious disease test management server 100 generate the certificate for infectious disease management or infectious disease safety for the community group (S530-1) and provides the generated certificate to the health manager of the community group S530-2 (S530).

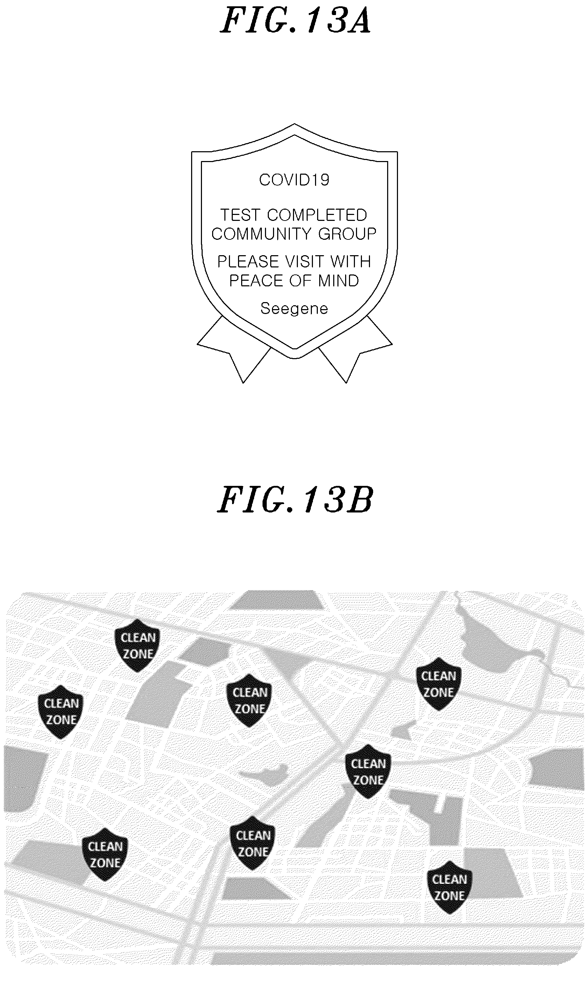

In Step S530, the certificate generated by the infectious disease test management server 100 is illustrated in an implementation example in FIG. 13A. As illustrated in FIG. 13A, the certificate includes the name of the infectious disease tested and the name of the community group.

Additionally, the certificate may further include an authentication period. For example, it is desirable that the infectious disease test be performed periodically, and in the certificate, a period from the time point of completion of the test to before the next infectious disease test may be set as an authentication period.

The infectious disease test management server 100 may display the certificate generated in Step S530 on the digital map provided by the infectious disease test management server 100 using the location information on the community group (S540).

Meanwhile, one implementation example of Step S540 may be displayed on the digital map provided by the infectious disease test management server 100. Another implementation example of Step S540 may provide the certificate and the certificate information including the location information on the community group to the external computing apparatus so that the certificate generated in Step S530 may be displayed on the digital map provided by the external computing apparatus.

The implementation example in which the infectious disease test management server 100 displays the certificate and the location of the community group on the digital map in Step S540 is illustrated in FIG. 13B. As illustrated in FIG. 13B, the infectious disease test management server 100 may display the community group that have acquired the certificates by performing the infectious disease test on the digital map provided to general users or customers.

In one implementation example of the present invention, the location of the community group that has acquired the certificate on the digital map may be displayed with text and/or an image such as, for example, "Clean Zone". The display on the digital map may refer to a general display method of a point of interest (POI).

The general users or customers may visit the community group that are safer from the infectious diseases through the certificate and location information displayed on the digital map. Accordingly, community groups that have obtained the certificates are expected to be recognized as safer from infectious diseases than other community groups, and thus more customers are expected to visit.

When the infectious disease test is repeated as an infectious disease test is performed periodically in the community group (S600), the infectious disease test for the community group may be re-performed from Step S200.

In accordance with an aspect of the present disclosure, there is provided a non-transitory computer-readable storage medium storing a computer program for processing a request for an infectious disease test for a community group and test result notification, the computer program configured to perform: generate a community group including a plurality of users; provide test information for infectious disease test to the plurality of users included in the community group; receive test results of the infectious disease test conducted on the plurality of users; generate an integrated test result of the community group using the test results; and provide the integrated test result to a health manager of the community group.

Since each component described in one embodiment of the present invention overlaps with the method for testing the community group for the infectious disease and notifying test results by the infectious disease test management server described with reference to FIGS. 1 to 13, description thereof is omitted.

In accordance with an aspect of the present disclosure, there is provided a computer program for processing a request for an infectious disease test for a community group and test result notification, stored in a non-transitory computer-readable storage medium, the computer program configured to perform: generate a community group including a plurality of users; provide test information for infectious disease test to the plurality of users included in the community group; receive test results of the infectious disease test conducted on the plurality of users; generate an integrated test result of the community group using the test results; and provide the integrated test result to a health manager of the community group.

Since each component described in one embodiment of the present invention overlaps with the method for testing the community group for the infectious disease and notifying test results by the infectious disease test management server described with reference to FIGS. 1 to 13, description thereof is omitted.

The invention and description in this specification illustrate preferred embodiments and are not limited to these embodiments. Those skilled in the art to which the present invention pertains may make various changes and modifications to the above embodiments without departing from the scope of the present invention, and the technical scope of the present invention encompasses all such changes and modifications.

### CROSS-REFERENCE TO RELEATED APPLICATION

This application claims priority to Korean Patent Application No. 10-2022-0094921, filed on July 29, 2022, under 35 U.S.C. § 119(a), the entire contents of which are incorporated herein by reference. Furthermore, this application claims priority in countries other than the United States for the same reason, and the entire contents thereof are also incorporated herein by reference.

## Claims

1. A method for processing a request for an infectious disease test for a community group and a test result notification in a computing apparatus using a memory, a processor, and one or more programs stored on the memory and configured to be executed by the processor, the method comprising:
generating, by the processor, a community group including a plurality of users;
providing, by the processor, a test information for the infectious disease test to the plurality of users included in the community group;
receiving, by the processor, test results of the infectious disease test conducted on the plurality of users;
generating, by the processor, an integrated test result of the community group using the test results; and
providing, by the processor, the integrated test result to a health manager of the community group.

2. The method of claim 1, further comprising:
providing, by the processor, to the health manager, an infectious disease comparison information obtained by comparing an infection index generated using the test results with an infection index generated using infectious disease test results for at least one of an administrative district including the community group and another community.

3. The method of claim 2, wherein the infection index includes at least one of an infection rate (positive rate) for the infectious disease, a basic reproduction number for the infectious disease, local safety index for the infectious disease, and contagion index for the infectious disease.

4. The method of claim 1, wherein the test information includes at least one selected from a group consisting of test reception request information, sampling method information, sampling available time information, sampling place information, test schedule information, questionnaire and medical institution reservation request information for the infectious disease test.

5. The method of claim 4, wherein the sampling method information relates to a method for collecting samples from the plurality of users for the infectious disease test, and
includes information that allows the plurality of users to select one of
i) the plurality of users visiting the medical institution,
ii) a sample collector visiting a location where the plurality of users are located, and
iii) the plurality of users directly collecting samples,
or information provided to the plurality of users about a pre-selected sampling method.

6. The method of claim 5, wherein, in the sampling method information,
i) when the plurality of users visiting the medical institution is selected, location information is received from each of the plurality of users and at least one of medical institution information adjacent to each of the received location information is provided to the plurality of corresponding users,
ii) when the sample collector visiting the location where the plurality of users are located is selected, location information is received from each of the plurality of users and each of the received location information is provided to the sample collector, and
iii) when the plurality of users directly collecting samples is selected, location information is received from each of the plurality of users and the received location information is set as a delivery location of a sampling kit.

7. The method of claim 4, wherein the test schedule information is a schedule for performing the infectious disease test that is applied to all of the community group or to each of the plurality of users.

8. The method of claim 7, wherein the schedule for performing the infectious disease test is at least one of a weekly schedule, a monthly schedule, a quarterly schedule, a semi-annual schedule, an annual schedule, and a set periodic schedule.

9. The method of claim 8, wherein the test information is provided to the plurality of users according to the test schedule information.

10. The method of claim 7, wherein the test schedule information further includes a notification schedule for the test results of the infectious disease test.

11. The method of claim 7, wherein the test schedule information further includes a schedule for another infectious disease test applied to the all of the community group or each of the plurality of users.

12. The method of claim 4, wherein in response to the provided test information, at least one selected from a group consisting of a reply to the test reception request information, a reply to the sampling method information, a reply to the sampling available time information, a reply to the questionnaire, and a reply to the medical institution reservation request is received from the plurality of users.

13. The method of claim 1, wherein in the receiving of the test results, the test results are received from a medical institution or test institution that performed the infectious disease test.

14. The method of claim 1, wherein the integrated test result includes an infectious disease statistical information for the community group generated using the test results corresponding to the plurality of users.

15. The method of claim 14, wherein the infectious disease statistical information is generated by the infectious disease test results and previous infectious disease test results to provide at least one of
i) information on a change in a positive rate of the infectious disease test over time,
ii) information on a positive rate prediction using the change in the positive rate, and
iii) information on a positive rate prediction using a cycle threshold (Ct) value of the infectious disease test over time.

16. The method of claim 14, wherein the integrated test result further includes the test results for the plurality of users.

17. The method of claim 1, further comprising:
generating, by the processor, return time point information when a previously positive user turns out negative for the infectious disease test and then returns to the community group.

18. The method of claim 17, wherein the return time point information is included in at least one of a test result for the user determined to be negative and the integrated test result.

19. The method of claim 1, further comprising:
providing, by the processor, a quarantine guideline stored for the community group to a corresponding user among the plurality of users.

20. The method of claim 1, further comprising:
providing, by the processor, to the health manager, a certificate for infectious disease management or safety for the community group when the test results and/or the integrated test results for the plurality of users belonging to the community group are not less than a threshold value.

21. The method of claim 20, further comprising:
displaying, by the processor, the generated certificate on a digital map provided by the computing apparatus using a location information on the community group.

22. The method of claim 20, further comprising:
providing, by the processor, to an external computing apparatus, a certificate information including the generated certificate and the location information on the community group so that the certificate is displayed on a digital map provided by the external computing apparatus.

23. The method of claim 1, the community group is generated using personal information on the plurality of users.

24. The method of claim 1, wherein the community group is in a plural number, and the plurality of users are included in two or more community groups.

25. The method of claim 1, further comprising:
prior to the providing of the test information, by the processor,
providing, to at least one of the plurality of users or the health manager, a plurality of infectious disease test items, for selection of an infectious disease test to be performed in the community group; and
receiving, from at least one of the plurality of users or the health manager, a selected test item from the plurality of infectious disease test items.

26. The method of claim 25, wherein the infectious disease test items include at least one selected from a group consisting of COVID-19, influenza, 2022 monkeypox, sexually transmitted infections, human papillomavirus infections, respiratory infections, gastrointestinal tract infections, tuberculosis, drug resistance, and meningitis.

27. The method of claim 1, wherein the infectious disease test is a molecular diagnostics test.

28. The method of claim 1, wherein the community group is at least one selected from a group consisting of an educational institution, a childcare institution, a postnatal care center, a company, a car sharing service, a transportation, a religious facility, a restaurant, a travel agency, a nursing home, a performance facility, a sports facility, and a shopping mall.

29. The method of claim 1, further comprising:
requesting, by the processor, consent from the plurality of users to provide the test results for the plurality of users to the community group.

30. The method of claim 1, wherein the infectious disease test for the community group is performed periodically.

31. A device for processing a request for an infectious disease test for a community group and a test result notification, the device comprising:
a memory configured to store at least one instruction; and
a processor;
wherein the at least one instruction is executed by the processor, to:
generate a community group including a plurality of users;
provide a test information for the infectious disease test to the plurality of users included in the community group;
receive test results of the infectious disease test conducted on the plurality of users;
generate an integrated test result of the community group using the test results; and
provide the integrated test result to a health manager of the community group.

32. The device of claim 31, wherein the at least one instruction executed by the processor further comprises providing, to the health manager, an infectious disease comparison information obtained by comparing an infection index generated using the test results with an infection index generated using infectious disease test results for at least one of an administrative district including the community group and another community.

33. The device of claim 32, wherein the infection index includes at least one of an infection rate (positive rate) for the infectious disease, a basic reproduction number for the infectious disease, local safety index for the infectious disease, and contagion index for the infectious disease.

34. The device of claim 31, wherein the test information includes at least one selected from a group consisting of test reception request information, sampling method information, sampling available time information, sampling place information, test schedule information, questionnaire and medical institution reservation request information for the infectious disease test.

35. The device of claim 34, wherein the sampling method information relates to a method for collecting samples from the plurality of users for the infectious disease test, and
includes information that allows the plurality of users to select one of
i) the plurality of users visiting the medical institution,
ii) a sample collector visiting a location where the plurality of users are located, and
iii) the plurality of users directly collecting samples,
or information provided to the plurality of users about a pre-selected sampling method.

36. The device of claim 35, wherein, in the sampling method information,
i) when the plurality of users visiting the medical institution is selected, location information is received from each of the plurality of users and at least one of medical institution information adjacent to each of the received location information is provided to the plurality of corresponding users,
ii) when the sample collector visiting the location where the plurality of users are located is selected, location information is received from each of the plurality of users and each of the received location information is provided to the sample collector, and
iii) when the plurality of users directly collecting samples is selected, location information is received from each of the plurality of users and the received location information is set as a delivery location of a sampling kit.

37. The device of claim 34, wherein the test schedule information is a schedule for performing the infectious disease test that is applied to all of the community group or to each of the plurality of users.

38. The device of claim 37, wherein the schedule for performing the infectious disease test is at least one of a weekly schedule, a monthly schedule, a quarterly schedule, a semi-annual schedule, an annual schedule, and a set periodic schedule.

39. The device of claim 38, wherein the test information is provided to the plurality of users according to the test schedule information.

40. The device of claim 37, wherein the test schedule information further includes a notification schedule for the test results of the infectious disease test.

41. The device of claim 37, wherein the test schedule information further includes a schedule for another infectious disease test applied to the all of the community group or each of the plurality of users.

42. The device of claim 34, wherein in response to the provided test information, at least one selected from a group consisting of a reply to the test reception request information, a reply to the sampling method information, a reply to the sampling available time information, a reply to the questionnaire, and a reply to the medical institution reservation request is received from the plurality of users.

43. The device of claim 31, wherein in the receiving of the test results, the test results are received from a medical institution or test institution that performed the infectious disease test.

44. The device of claim 31, wherein the integrated test result includes an infectious disease statistical information for the community group generated using the test results corresponding to the plurality of users.

45. The device of claim 44, wherein the infectious disease statistical information is generated by the infectious disease test results and previous infectious disease test results to provide at least one of
i) information on a change in a positive rate of the infectious disease test over time,
ii) information on a positive rate prediction using the change in the positive rate, and
iii) information on a positive rate prediction using a cycle threshold (Ct) value of the infectious disease test over time.

46. The device of claim 44, wherein the integrated test result further includes the test results for the plurality of users.

47. The device of claim 31, wherein the at least one instruction executed by the processor further comprises generating return time point information when a previously positive user turns out the negative for the infectious disease test result and then to the community group.

48. The device of claim 47, wherein the return time point information is included in at least one of a test result for the user determined to be negative and the integrated test result.

49. The device of claim 31, wherein the at least one instruction is executed by the processor further comprises providing a quarantine guideline stored for the community group to a corresponding user among the plurality of users.

50. The device of claim 31, wherein the at least one instruction executed by the processor further comprises providing, to the health manager, a certificate for infectious disease management or safety for the community group when the test results and/or the integrated test results for the plurality of users belonging to the community group are not less than a threshold value.

51. The device of claim 50, wherein the at least one instruction executed by the processor further comprises displaying the generated certificate on a digital map provided by the computing apparatus using a location information on the community group.

52. The device of claim 50, wherein the at least one instruction executed by the processor further comprises providing, to an external computing apparatus, a certificate information including the generated certificate and the location information on the community group so that the certificate is displayed on a digital map provided by the external computing apparatus.

53. The device of claim 31, wherein in the generating of the community group, the community group is generated using personal information on the plurality of users.

54. The device of claim 31, wherein the community group is in plural number, and the plurality of users are included in two or more community groups.

55. The device of claim 31, wherein the at least one instruction executed by the processor further comprises,
prior to the providing of the test information,
providing, to at least one of the plurality of users or the health manager, a plurality of infectious disease test items, for selection of an infectious disease test to be performed in the community group; and
receiving, from at least one of the plurality of users or the health manager, a selected test item from the plurality of infectious disease test items.

56. The device of claim 55, wherein the infectious disease test items include at least one selected from a group consisting of COVID-19, influenza, 2022 monkeypox, sexually transmitted infections, human papillomavirus infections, respiratory infections, gastrointestinal tract infections, tuberculosis, drug resistance, and meningitis.

57. The device of claim 31, wherein the infectious disease test is a molecular diagnostics test.

58. The device of claim 31, wherein the community group is at least one selected from a group consisting of an educational institution, a childcare institution, a postnatal care center, a company, a car sharing service, a transportation, a religious facility, a restaurant, a travel agency, a nursing home, a performance facility, a sports facility, and a shopping mall.

59. The device of claim 31, wherein the at least one instruction executed by the processor further comprises requesting consent from the plurality of users to provide the test results for the plurality of users to the community group.

60. The device of claim 31, wherein the infectious disease test for the community group is performed periodically.

61. A non-transitory computer-readable storage medium storing a computer program for processing a request for an infectious disease test for a community group and a test result notification, the computer program configured to perform:
generate a community group including a plurality of users;
provide a test information for the infectious disease test to the plurality of users included in the community group;
receive test results of the infectious disease test conducted on the plurality of users;
generate an integrated test result of the community group using the test results; and
provide the integrated test result to a health manager of the community group.

62. A computer program for processing a request for an infectious disease test for a community group and a test result notification, stored in a non-transitory computer-readable storage medium, the computer program configured to perform:
generate a community group including a plurality of users;
provide a test information for the infectious disease test to the plurality of users included in the community group;
receive test results of the infectious disease test conducted on the plurality of users;
generate an integrated test result of the community group using the test results; and provide the integrated test result to a health manager of the community group.
